# EUROPEAN PATENT SPECIFICATION

(11) **EP 3 080 353 B1**
(45) Date of publication and mention of the grant of the patent: **26.01.2022**
(21) Application number: 14821093.3
(22) Date of filing: 11.12.2014
(51) Int. Cl.: D21C 11/00, D21C 11/04

(54) **PROCESS FOR LIGNIN PURIFICATION AND ISOLATION**
VERFAHREN ZUR LIGNINREINIGUNG UND -ISOLIERUNG
PROCÉDÉ DE PURIFICATION ET D'ISOLEMENT DE LIGNINE

(30) Priority: 12.12.2013 EP 13196954
(43) Date of publication of application: 19.10.2016
(73) Proprietor: Annikki GmbH, 8074 Raaba-Grambach (AT)
(72) Inventor: DYBOV, Alexander, 8073 Seiersberg-Pirka (AT); MATEJKA, Julia, 8062 Kumberg (AT)
(74) Representative: Nemec, Harald
(86) International application number: PCT/EP2014/077450
(87) International publication number: WO 2015/086772

(56) References cited:
- EP-A1- 0 224 721
- EP-A2- 0 140 226
- WO-A1-00/17444
- WO-A1-2008/079072
- WO-A1-2012/117161
- WO-A2-2012/054947
- CA-A1- 2 026 585
- US-A- 2 445 838
- US-A- 4 764 596
- US-A1- 2004 244 925

## Description

The present invention relates to a process for lignin purification and isolation via precipitation from an alcoholic basic lignin solution.

### Background of the invention

Alkali organosolv pulping is an attractive technique to selectively isolate lignin from the lignocellulosic material. The method is capable to deliver high value cellulose which is comparable and in some aspects better than that obtained by kraft process (e.g. DE 4103572 A1).

In another aspect the lignin may be separated from the lignocellulosic material without applying any sulfur containing reagent. In contrast to kraft or sulfite pulping processes, wherein the lignin is burned for energy recovery the lignin isolated by an alkali organosolv method may be a high value product since the contamination by toxic sulfur compound and consequent change in the lignin structure are avoided. To pursue biorefinary concepts the lignin should be recovered from the organosolv lignin solution and utilized in the production of bio-based polymeric material.

However, the lignin recovery from the alkali solution in the organosolv process (alkali organosolv solution) may face problems, in particular in the alcohol recovery stage due to a strong tendency of said lignin solution to build foam while alcohol is evaporated. The addition of anti-foaming reagent such as surfactants may partially prevent the foam formation. But the use of anti-foaming reagents increases the process costs and strongly contaminates the precipitated lignin.

In another aspect, for successful lignin recovery the alkali organosolv solution may be neutralized to cause lignin precipitation. The neutralization often leads to the formation of inorganic salts. These by-products remain in the aqueous solution after lignin isolation and require utilization which may be tedious due to high solubility of those salts in water.

For delignification of non-wood material the recovery of the cooking chemicals may be found difficult due to the presence of silicates in the plant material. In particular, it is referred to the alkaline pulping as the silica is prone to be dissolved in alkaline media (e.g. EP1115944 A1). The dissolved silicates settle on the hot surfaces of evaporation equipment in the chemical circulation unless a method of silica separation from the spent liquor is provided. Moreover, the silicates may increase the ash content (inorganic contamination) of the target lignin. Such contaminations are crucial for some application (e.g. carbon fiber) and it is very difficult to remove it.

### State of the art

There are some known methods for recovery of dissolved lignin from pulping liquors. No one, however, refers to the problems mentioned above.

In US 4,764,596 a method for lignin recovery from black liquor produced by pulping wood at high temperatures and pressures with an aqueous aliphatic alcohol solvent is disclosed. The lignin is precipitated by diluting the black liquor with water and an acid to form a solution with a pH of less than 3 and an alcohol content of less than about 30%. The precipitated lignin is obtained in a form of a powder after drying and could be used without further significant processing.

In AT 510 812 a process for lignin isolation is described, wherein an acid is added to a basic alcoholic solution of lignin for lignin precipitation. The precipitated lignin is separated, and ethanol is evaporated from the mother liquid after lignin separation whereby further lignin fractions could be obtained.

From EP 420 771 A1 a method for isolating lignin from a solvent pulping liquor containing lignin and a lower alcohol solvent is known, comprising the steps of: (1) continuously adding solvent pulping liquor to a stripping column, (2) heating the pulping liquor in the stripping column to produce an alcohol-rich overhead vapor stream and a residual slurry comprising lignin, (3) continuously withdrawing the overhead vapor stream from the stripping column, and (4) isolating the residual slurry. The lignin is precipitated from the residual slurry and separated.

In CA 2,026,585 A1 a method is disclosed, wherein lignin is isolated from acidic solvent pulping liquors by adjusting the pH of a solvent pulping liquor to a pH of greater than 3.0 but less than or equal to 7.0 which results in the formation of an easily filterable lignin precipitate when a portion of the lower alcohol is evaporated from the solvent pulping liquor. The lower alcohol may be evaporated by direct contact heating in a stirred tank without the formation of a tarry precipitate. The particular invention refers to a method to isolate the lignin from acidic solvent pulping solution. In particular, a base should be added to the solution for pH adjusting.

### Detailed description of the present invention

The present invention provides a method to overcome problems mentioned above. A method is provided for separating inorganic substances from a lignin solution from the organosolv process, thus allowing an easy separation of lignin which is practically free from inorganic salts from said solution - e.g. the isolated lignin was found to be practically ash free lignin. According to the present invention furthermore alcohol may be recovered from said lignin solution without foam formation which consequently may lead to an enormous increase in the alcohol recovery rate.

In sum, the present invention thus provides a method to separate lignin from an alcoholic basic (alkali) lignin solution, comprising steps to avoid foaming during ethanol recovery and to remove and recover inorganic salts formed during lignin precipitation.

The present invention is defined by the process steps of the current claim 1.

An alcoholic basic lignin solution as a starting material in a process of the present invention may be obtained by contacting lignocellulosic material with an aqueous solution comprising at least one base and one low aliphatic alcohol.

The chemical nature of the base is not of great importance and a base may be chosen from known bases, e.g. inorganic bases, such as hydroxides, e.g. sodium hydroxide, potassium hydroxide, calcium hydroxide; carbonates, e.g. sodium carbonate, potassium carbonate, calcium carbonate, ammonia, and individual mixtures from two or more of such bases. In one embodiment of the present invention the base is sodium hydroxide.

An alcohol useful in a process of the present invention is preferably a C₁-C₄ alcohol, e.g. including methanol, ethanol, n-propanol, isopropanol, n-butanol, isobutanol and t-butanol, or individual mixtures from two or more of such alcohols, most preferably methanol or ethanol.

In one embodiment of the present invention sodium hydroxide as a base and ethanol as an alcohol is used to obtain an alcoholic basic lignin solution.

According to the present invention alcohol, e.g. ethanol may be added to the to the alcoholic lignin solution to increase the alcohol concentration so that the alcohol concentration of said solution is sufficient that at least 80%, preferably 90% most preferably 100% silicate present in said lignin solution are precipitated. For that e. g. an alcohol concentration of 50% by weight and more, preferably, 55% by weight and more, most preferably 60% by weight and more, e.g. from 50% to 80%, such as 55% to 70% by weight is adjusted in the alcoholic lignin solution, We have surprisingly found that such an increase in alcohol concentration results in completeness of inorganic salt precipitation from said alcoholic lignin solution.

In one embodiment of the present invention the alcohol concentration may be increased by adding pure alcohol, or by adding a mixture comprising alcohol and water with an alcohol concentration of 70% and more, preferably 80% and more, most preferably 90% and more, e.g. 70% to 95%. In one particular embodiment a mixture of alcohol and water comprising lignin with an alcohol concentration of 70% and more may be added to alcoholic lignin solution.

In one specific aspect of the present invention the contact of said aqueous alcohol solution with lignocellulosic material to obtain an alcoholic basic lignin solution is carried out at elevated temperatures, e.g. in the range from 50 to 210°C, e.g. for a time sufficient to extract at least 50%, preferably 60%, most preferably 75% of the lignin present in the starting lignocellulosic material.

In the process of the present invention is sulfuric acid added to the alcoholic aqueous lignin solution in step (a). The acid is added in step (a) in an amount sufficient to drop the pH of said lignin solution at least to pH 9 or below, preferably, to pH 7 or below, most preferably to pH 5 or below, such as a pH-range of 2 to 9, e.g. 5 to 7, whereby an alcoholic lignin solution is formed.

According to the present invention an inorganic precipitate forms after the addition of an acid to the alcoholic basic lignin solution. In one particular embodiment, the inorganic salt comprises sodium sulfate, e.g. if sulfuric acid is used as an acid in step (a). We have unexpectedly observed that the inorganic precipitate contains a high amount of silicates extracted from straw while pulping. It was surprisingly found that in a process of the present invention the precipitate isolated in step (b) comprises at least 50%, e.g. 75%, up to practically 100% of the silicates dissolved in the solution while pulping.

According to the present invention, the precipitate formed after addition of an acid to the alcoholic basic lignin solution in step (a) is separated from said solution in step (b) by an appropriate solid-liquid separation method, e.g. a known separation method, such as sedimentation, filtration, centrifugation, flotation, flocculation, hydro cyclonic separation, or any combination and/or any modification thereof, preferably by filtration.

A lignin solution is obtained which is practically free from inorganic substances.

From said alcoholic lignin solution lignin may be precipitated via alcohol removal.

In one embodiment of the present invention an alcohol alkali solution obtained after the removal of the precipitate in step (b) is concentrated, e.g. via a membrane concentration method, e.g. via nano-filtration. The nano-filtration may be conducted as appropriate, e.g. using a suitable membrane that allows lignin retention.

In one embodiment of the present invention alcohol is removed from the alcoholic acidic lignin solution in step (c) by evaporation or distillation, or a combination thereof, preferably by flash evaporation, whereby a lignin suspension and an alcohol rich vapor fraction are formed. Preferably the evaporation is carried out until an ethanol concentration in the lignin suspension of 15% by weight and below, more preferably of 5% by weight and below, e.g. 0.1 to 15% by weight is achieved. In one embodiment of the present invention, the alcohol rich vapor fraction obtained during evaporation is condensed to obtain a liquid fraction comprising alcohol.

According to present invention the evaporation may be carried out in an evaporator, e.g. forced circulation evaporator, draft-tube-baffle evaporator or stirred-tank evaporator.

In one particular embodiment of the present invention the liquid fraction comprising alcohol is further fractionated, whereby a liquid fraction which is still further enriched with alcohol is obtained.

According to the present invention, lignin may be separated in step (e) from said lignin suspension as appropriate, e.g. by a solid-liquid separation method, such as sedimentation, filtration, centrifugation, flotation, flocculation, hydro cyclonic separation, any combination and/or any modification thereof, preferably by filtration or centrifugation.

In one embodiment of present invention the pH of the lignin suspension is adjusted before lignin separation to 5 and below, preferably to 3 and below, most preferably to 2 and below, e.g. the pH is adjusted to a pH from 1 to 5, such as 1 to 3.

The pH adjustment may be performed by addition of an acid, e.g. inorganic acid, such as nitric acid, sulfuric acid, hydrochloric acid, phosphoric acid, carbon dioxide, sulfur dioxide, sulfur trioxide or any combination of two or more of such acids. In one embodiment of the present invention sulfuric acid is used for pH adjustment. We have surprisingly found that such pH adjustment of the lignin suspension may result in an increase of the lignin yield separated from the alcoholic basic lignin solution.

Lignin separated from the lignin suspension according to the present invention is preferably further treated, e.g. washed and dried.

In one embodiment of the present invention, the lignin washing is performed with an acidic, e.g. aqueous, solution, having a pH from 1 to 3. An appropriate acid includes inorganic acids, such as nitric acid, sulfuric acid, hydrochloric acid, phosphoric acid, carbon dioxide, sulfur dioxide, sulfur trioxide or any individual combination of two or more of such acids. In one particular embodiment of the present invention, the acidic solution for lignin washing comprises sulfuric acid. Unexpectedly, we have observed that washing the lignin with an acidic solution reduces the lignin loss during washing compared with neutral washing.

In one embodiment of the present invention the lignin washed with an acidic solution as described above is further washed with water. In one particular embodiment of the present invention the washing with water is conducted until the pH of the washing solution is 4 and more, preferably 5 and more, most preferably neutral, e.g. the washing is conducted until a pH of 4 to 7 of the washing solution is achieved.

Separated lignin obtained according to the present invention is dried, optionally after washing. Drying may be carried out at elevated temperatures, e.g. up to 60°C, preferably below 50°C, most preferably below 40°C, e.g. in a range of 30°C to 60°C. In one embodiment of the present invention the drying of the separated lignin is performed in vacuum. The drying should last for a period of time which is sufficient to obtain lignin with a water contain of 10% and less, preferably 5% and less, most preferably 1% and less, e.g. 0.1% to 10%.

We have furthermore surprisingly observed that the addition of sulfuric acid to the alcoholic basic lignin solution reduces the tendency of said solution to build foam. If a sufficient amount of an acid, e.g. an amount sufficient to drop the pH of said solution to 2 to 9 and below, e.g. 5 to 7, is added to the solution, the foaming can be practically completely avoided during alcohol evaporation.

In another aspect the present invention provides a method for avoiding foaming during alcohol recovery from an alcoholic mixture comprising lignin, which method is characterized in that a pH of 2 to 9 of the alcoholic mixture is adjusted during alcohol recovery.

### Example 1

### Preparation of alcohol alkali lignin solution.

1500 g of wheat straw (21% lignin content, particle size ≈ 2 cm) were contacted with a solution comprising water and 52% by weight of EtOH in the presence of 10% NaOH (related to the dry mass of the straw) at 70°C for 18 hours. The consistency of the reaction mixture was 10% by weight at the beginning. The lignin solution was separated by pressing the reaction suspension at room temperature. The ash content of the solution (determined according to DIN 54370) was 1.45 %wt.

### Lignin isolation.

Concentrated sulfuric acid (98% wt.) was added to 1 kg of the lignin solution until the pH of the solution was adjusted to 5. The precipitate formed was filtered off and dried in vacuum. The yield of the precipitate was 7.6 g. The acid insoluble ash content of the precipitate (determined according to TAPPI T 244 cm-11) was found to be 3.0% wt.

Ethanol was evaporated from the filtrate under vacuum (below 200 mbar). No foaming was observed during evaporation. The evaporation was continued until the ethanol concentration was below 1%. The formed lignin suspension was acidified with concentrated sulfuric acid to a pH of 2. The lignin suspension was centrifuged, lignin was isolated, washed with diluted sulfuric acid solution (pH = 2) and then two times with water. The obtained lignin was dried in vacuum at 35°C and was found to be practically free from inorganic substances (salts).

### Example 2

A lignin solution was prepared as it was described in Example 1.

Concentrated sulfuric acid (98% wt.) was added to 1 kg of the lignin solution and a pH of the solution of 5 was adjusted. To the mixture obtained ethanol was added until an ethanol concentration in the solution of 60 % by weight was adjusted. The precipitate formed was filtered off and dried in vacuum. The yield of the precipitate was 9.6 g. The acid insoluble ash content of the precipitate (determined according to TAPPI T 244 cm-11) was found to be 3.0% wt.

Ethanol was evaporated from the filtrate under vacuum (below 200 mbar). No foaming was observed during evaporation. Evaporation was continued until the ethanol concentration ion the evaporation residue was below 1%. The formed lignin suspension was acidified with concentrated sulfuric acid to pH 2. The lignin suspension obtained was centrifuged and lignin was separated. The lignin obtained was washed with diluted sulfuric acid (pH = 2) and then two times with water. The lignin obtained was dried in vacuum at 35 °C and was found to be practically free from inorganic substances (salts).

## Claims

1. A method for separating lignin from an alcoholic basic lignin solution, comprising the steps
(i) adjusting an alcohol content of 50% to 80% by weight in the alcoholic mixture comprising lignin
(a) adding sulfuric acid to said solution whereby an alcoholic lignin solution is formed,
(b) separating a precipitate comprising an inorganic precipitate, particularly comprising a silicate, from said solution.
(c) removing alcohol from said solution,
(d) optionally adjusting the pH of the mixture obtained in step (c), and
(e) separating lignin from said mixture.

2. A process according to claim 1 , wherein in step (a) a pH of 2 to 9, particularly of 5 to 7 is adjusted.

3. A method of any one of claims 1 to 2, wherein said alcoholic basic lignin solution is obtained by pulping a lignocellulosic material by contacting the material with an aqueous solution comprising at least one low aliphatic alcohol, in particular C₁-C₄ alcohol, in particular ethanol or methanol, and an inorganic base, particularly sodium hydroxide, at elevated temperatures for a time sufficient to extract at least 50%, preferably at least 60%, most preferably 75% of the initial lignin from said lignocellulosic material.

4. A method according to any one of claims 1 to 3, wherein such an amount of alcohol, particularly ethanol or methanol is in, or is added to the alcoholic lignin solution that the alcohol concentration of said solution becomes appropriate to precipitate at least 80%, particularly 90%, particularly 100% of silicate present in said lignin solution.

5. A method according to any one of claims 1 to 4, wherein said precipitate in step (b) comprises sodium sulfate or a mixture of sodium sulfate and silicate.

6. A method according to any one of claim 1 to 5, wherein the alcohol concentration in step (c) is adjusted to an amount of 15% alcohol by weight and below, in particular to 5% by weight and below, particularly 0.1 to 15% by weight.

7. A method according to any one of claim 1 to 5, wherein the pH of the lignin suspension in step (d) is adjusted to pH 1 to 5 by addition of an inorganic acid, in particular sulfuric acid, to said lignin suspension.

8. A method according to any one of claim 1 to 7, wherein said lignin in step (e) is separated from the lignin suspension by a solid-liquid separation method, in particular by centrifugation or filtration.

9. A method according to any one of claim 1 to 8, wherein said lignin which is separated from said lignin suspension in step (e) is washed and dried after separation.

10. A method according to claim 8, wherein said lignin which is separated from said lignin suspension in step (e) is washed with an aqueous acidic solution, optionally followed by water.

## Patentansprüche

1. Verfahren zum Heraustrennen von Lignin aus einer alkoholischen basischen Ligninlösung, umfassend die folgenden Schritte:
(i) Einstellen eines Alkoholgehalts von 50 bis 80 Gew.-% in dem alkoholischen Gemisch, umfassend Lignin,
(a) Hinzufügen von Schwefelsäure zu der Lösung, wodurch eine alkoholische Ligninlösung gebildet wird,
(b) Abtrennen eines Niederschlags, umfassend einen anorganischen Niederschlag, insbesondere umfassend ein Silikat, von der Lösung,
(c) Entfernen von Alkohol aus der Lösung,
(d) gegebenenfalls Einstellen des pH-Werts des in Schritt (c) erhaltenen Gemisches, und
(e) Heraustrennen von Lignin aus dem Gemisch.

2. Vorgang gemäß Anspruch 1, wobei in Schritt (a) ein pH-Wert von 2 bis 9, insbesondere von 5 bis 7, eingestellt wird.

3. Verfahren gemäß einem der Ansprüche 1 bis 2, wobei die alkoholische basische Ligninlösung durch Aufschluss eines lignocellulosischen Materials erhalten wird, indem das Material mit einer wässrigen Lösung, die mindestens einen niederen aliphatischen Alkohol, insbesondere C₁-C₄-Alkohol, insbesondere Ethanol oder Methanol, und eine anorganische Base, insbesondere Natriumhydroxid, umfasst, bei erhöhten Temperaturen für eine ausreichende Zeit in Kontakt gebracht wird, um mindestens 50%, vorzugsweise mindestens 60%, am meisten bevorzugt 75% des anfänglichen Lignins aus dem lignocellulosischen Material zu extrahieren.

4. Verfahren gemäß einem der Ansprüche 1 bis 3, wobei eine solche Menge an Alkohol, insbesondere Ethanol oder Methanol, in der alkoholischen Ligninlösung vorhanden ist oder zu dieser hinzugefügt wird, dass die Alkoholkonzentration der Lösung geeignet wird, um mindestens 80%, insbesondere 90%, insbesondere 100% des in der Ligninlösung vorhandenen Silikats auszufällen.

5. Verfahren gemäß einem der Ansprüche 1 bis 4, wobei der Niederschlag in Schritt (b) Natriumsulfat oder ein Gemisch aus Natriumsulfat und Silikat umfasst.

6. Verfahren gemäß einem der Ansprüche 1 bis 5, wobei die Alkoholkonzentration in Schritt (c) auf eine Menge von 15 Gew.-% Alkohol und weniger, insbesondere auf 5 Gew.-% und weniger, insbesondere auf 0,1 bis 15 Gew.-%, eingestellt wird.

7. Verfahren gemäß einem der Ansprüche 1 bis 5, wobei der pH-Wert der Ligninsuspension in Schritt (d) durch Hinzufügung einer anorganischen Säure, insbesondere Schwefelsäure, zu der Ligninsuspension auf pH 1 bis 5 eingestellt wird.

8. Verfahren gemäß einem der Ansprüche 1 bis 7, wobei das Lignin in Schritt (e) durch ein Fest-Flüssig-Trennverfahren, insbesondere durch Zentrifugation oder Filtration, von der Ligninsuspension getrennt wird.

9. Verfahren gemäß einem der Ansprüche 1 bis 8, wobei das Lignin, das in Schritt (e) von der Ligninsuspension getrennt wird, nach der Trennung gewaschen und getrocknet wird.

10. Verfahren gemäß Anspruch 8, wobei das Lignin, das in Schritt (e) von der Ligninsuspension getrennt wird, mit einer wässrigen sauren Lösung, gegebenenfalls gefolgt von Wasser, gewaschen wird.

## Revendications

1. Procédé de séparation de lignine à partir d'une solution de lignine basique alcoolique, comprenant les étapes consistant à
(i) ajuster une teneur en alcool de 50 % à 80 % en poids dans le mélange alcoolique comprenant de la lignine,
(a) ajouter de l'acide sulfurique à ladite solution, de telle sorte qu'une solution de lignine alcoolique soit formée,
(b) séparer un précipité comprenant un précipité inorganique, en particulier comprenant un silicate, à partir de ladite solution,
(c) éliminer l'alcool à partir de ladite solution,
(d) ajuster facultativement le pH du mélange obtenu dans l'étape (c), et
(e) séparer la lignine à partir dudit mélange.

2. Procédé selon la revendication 1 , dans lequel à l'étape (a), un pH de 2 à 9, en particulier de 5 à 7, est ajusté.

3. Procédé selon l'une quelconque des revendications 1 à 2, dans lequel ladite solution de lignine basique alcoolique est obtenue par réduction en pâte d'un matériau lignocellulosique par mise en contact du matériau avec une solution aqueuse comprenant au moins un alcool faiblement aliphatique, en particulier un alcool en C₁-C₄, en particulier de l'éthanol ou du méthanol, et une base inorganique, en particulier de l'hydroxyde de sodium, à des températures élevées pendant un temps suffisant pour extraire au moins 50 %, de préférence au moins 60 %, de la manière la plus préférée 75 % de la lignine initiale à partir dudit matériau lignocellulosique.

4. Procédé selon l'une quelconque des revendications 1 à 3, dans lequel une quantité d'alcool, en particulier d'éthanol ou de méthanol, est dans ou est ajoutée à la solution de lignine alcoolique de telle sorte que la concentration en alcool de ladite solution devient appropriée pour précipiter au moins 80 %, en particulier 90 %, en particulier 100 % de silicate présent dans ladite solution de lignine.

5. Procédé selon l'une quelconque des revendications 1 à 4, dans lequel ledit précipité de l'étape (b) comprend du sulfate de sodium ou un mélange de sulfate de sodium et de silicate.

6. Procédé selon l'une quelconque des revendications 1 à 5, dans lequel la concentration en alcool dans l'étape (c) est ajustée à une quantité de 15 % d'alcool en poids et moins, en particulier à 5 % en poids et moins, en particulier de 0,1 à 15 % en poids.

7. Procédé selon l'une quelconque des revendications 1 à 5, dans lequel le pH de la suspension de lignine dans l'étape (d) est ajusté à un pH de 1 à 5 par ajout d'un acide inorganique, en particulier d'acide sulfurique, à ladite suspension de lignine.

8. Procédé selon l'une quelconque des revendications 1 à 7, dans lequel ladite lignine dans l'étape (e) est séparée de ladite suspension de lignine par un procédé de séparation solide-liquide, en particulier par centrifugation ou filtration.

9. Procédé selon l'une quelconque des revendications 1 à 8, dans lequel ladite lignine qui est séparée de ladite suspension de lignine dans l'étape (e) est lavée et séchée après séparation.

10. Procédé selon la revendication 8, dans lequel ladite lignine qui est séparée de ladite suspension de lignine dans l'étape (e) est lavée avec une solution acide aqueuse, facultativement suivie d'eau.
